# EUROPEAN PATENT APPLICATION

(11) **EP 1 555 030 A1**
(43) Date of publication of application: **20.07.2005**
(21) Application number: 03748569.5
(22) Date of filing: 24.09.2003
(51) Int. Cl.: A61K 38/17, A61K 38/22, A61K 45/00, A61K 47/42, A61P 9/00, A61P 9/10, A61P 43/00

(54) **SUSTAINED RELEASE PREPARATION FOR TREATING CORONARY STENOSIS OR OBSTRUCTION**

(30) Priority: 25.09.2002 JP 2002279058
(71) Applicant: Medgel Corporation, Kyoto-shi, Kyoto 612-8043 (JP)
(72) Inventor: TABATA, Yasuhiko, Uji-shi, Kyoto 611-0024 (JP); KOMEDA, Masashi Gosho-nishi Urban Life 303, Kamigyo-ku Kyoto-shi, Kyoto 602-8024 (JP)
(74) Representative: VOSSIUS & PARTNER
(86) International application number: PCT/JP2003/012139
(87) International publication number: WO 2004/028556

(57) **Abstract**

A pharmaceutical composition for therapy of coronary artery narrowing or blockage, which comprises a sustained release preparation containing an angiogenesis factor or a gene encoding the same and a gelatin hydrogel is disclosed. Examples of the angiogenesis factor which may be used include e.g., bFGF and other FGFs, VEGF, HGF, PDGF, TGF, angiopoietin, HIF, cytokine, chemokine, adrenomeduline and the like.

## Description

### [Technical Field]

The present invention relates to a sustained release preparation for therapy of coronary artery narrowing or blockage. More particularly, the invention relates to a sustained release preparation containing an angiogenesis factor for use in therapy of narrowing or blockage of a coronary artery that is a nutrient artery of heart, and a novel therapeutic method of coronary artery narrowing or blockage using the same.

### [Background Art]

In the field of cardiac surgery, cardiac angina caused by narrowing of coronary artery, i.e., myocardial ischemia, and myocardial infarction caused as a result of progression of the lesion are serious diseases, the early detection and appropriate therapy of which may affect the patient' life. Particularly, myocardial infarction is one of three major causes of death of adults in Japan, and it is an urgent need to develop more effective therapy thereof. Even though progression of these diseases can be retarded by administration of an antihypertensive drug, a circulation improving agent such as a vasodilating agent, it is merely an auxiliary means, but does not become a basic remedy. As a direct therapeutic method on a blood vessel that was narrowed or blocked, there is a medical catheter therapy in which the coronary artery lesioned part is mechanically pushed and stretched. However, this method is accompanied by a problem of renarrowing, and there exists a limitation of the therapy when severe arteriosclerosis is found. In such a case, the remaining therapeutic method is a coronary artery bypass surgery in which other blood vessel to be a source of blood supply is surgically connected to a site that is more distal to the lesioned site, whereby producing a novel blood route (bypass). However, anastomosis of fine blood vessels having a diameter of less than 1 mm is technically difficult. Alternatively, even though the bypass is successfully produced, it follows the fate of blockage in the early stage due to the difference in diameters of blood vessel and insufficient amount of the blood flow. Also, the bypass surgery is applicable to a blood vessel having a diameter of 1.5 mm or greater, however, when other narrowing is present in its peripheral part or blenched blood vessel (particularly, when the diameter thereof being so small), effect of the bypass is reduced, and the patency also becomes inferior.

In severe cardiac angina patients having narrowed lesions in multiple coronary arteries in a generalized manner, any effective therapeutic method is not present at this moment, therefore, extremely bad prognosis is obliged. It becomes lethal when multiple organ failure is coincidentally caused which is accompanied by ischemic heart failure. In an attempt to pave the way out of this critical situation, therapies in which various angiogenesis factors are intramuscularly injected into a myocardial region or infused into a coronary artery in such cases where fine blood vessels are perfused to which medical (catheter intervention such as PCI or PTCA stent) or surgical (AC bypass or CABG, off-pump CABG, OPCAB or the like) therapies can not be adapted at the present stage. In addition, therapies in which advance of collateral circulation within cardiac muscle is accelerated by opening a pore from the outside of the heart with a laser have been initiated, although their effects are still unsatisfactory. In either process, the newly formed blood vessel merely expands the communication of originally residing intramyocardial blood vessels, therefore, amount of the blood thereby increased is insufficient for the recovery of cardiac functions with reduced contractive force. Accordingly, in this technical field, development of a novel therapeutic method for supplying sufficient blood to a coronary artery has been desired.

Prior art document information relating to the invention includes the followings: W094/27630; JP-A-2001-316285; and JP-A-2002-145797.

An object of the invention is to provide a novel method for the therapy of coronary artery narrowing or blockage by stimulating angiogenesis in a myocardial region, as well as a preparation for use in this method.

### [Disclosure of the Invention]

The present inventors found that a sustained release preparation of an angiogenesis factor produced using a gelatin hydrogel exerts a prominent therapeutic effect in a model rabbit of myocardial infarction. Thus, the present invention was accomplished. More specifically, the invention provides a pharmaceutical composition for use in therapy of coronary artery narrowing or blockage comprising a sustained release preparation containing an angiogenesis factor or a gene encoding the same and a gelatin hydrogel. The invention also provides a method for treating of coronary artery narrowing or blockage which comprises administering to a patient a sustained release preparation containing an angiogenesis factor or a gene encoding the same and a gelatin hydrogel. Preferably, the angiogenesis factor is bFGF.

By inserting a sheet prepared by impregnating bFGF into a gelatin hydrogel to be sandwiched between a myocardial ischemic site and a gastroepiploic artery having a large number of branches and allowing for sustained release according to the invention, an angiogenetic effect is achieved, and a new blood vessel is induced from a blood vessel outside of the heart thereby improving the blood flow to lead to expectation of remedy of ischemia. More specifically, use of the sustained release preparation of the invention induces a blood vessel from outside of the heart, therefore, connection of bypass without using a conventional anastomosis process for the aforementioned fine blood vessel (with extremely bad results found for blood vessels having a diameter of 1.5 mm or less, particularly, 1 mm or less), i.e., so-called "bio-bypass (Bio-bypass) " formation has come to be enabled. The invention is particularly useful as a therapeutic agent for severe ischemic heart diseases.

The sustained release preparation of the invention is useful for employing in a bypass (anastomosis) of a gastroepiploic artery or a greater omentum with a coronary artery or a branched or capillary vessel thereof, a bypass (anastomosis) of a left or right internal thoracic artery and a coronary artery or a branched or capillary vessel thereof, and a bypass (anastomosis) of a left or right radial artery and a coronary artery or a branched or capillary vessel thereof.

Greater omentum which is a connective tissue that is rich in capillary vessels and is present such that it wraps around the intestinal tract in the abdominal cavity uses the right gastroepiploic artery as a principal nutrient artery. This right gastroepiploic artery has been generally used as a graft in coronary artery bypass surgeries (novel root for supplying blood), and is a blood vessel having a high quality that is excellent in remote phase patency. In a preferred embodiment of the invention, a sustained release growth factor is positioned such that it is inserted to be sandwiched between the heart and the right gastroepiploic artery including greater omentum, and thus, a bio-bypass is formed to lead to budding of a blood vessel from this principal artery toward the cardiac muscle. Increase in blood flow due to bio-bypass is believed to be greater than increase in blood flow just due to development of intramyocardial collateral circulation. Therefore, high therapeutic effect is expected. Accordingly, therapeutic adaptation of a severe ischemic heart which has been hitherto deemed to be insusceptible of any medical or surgical treatment is enlarged, and thus, greatly good outcome may be possibly brought to patients suffering from cardiac failure or anginal pain. In particular, it can be also effective for patients suffering from ischemic cardiomyopathy among patients to which heart transplants have been adapted.

In addition, according to the invention, applications in other artery graft materials (internal thoracic arteries having the highest quality among blood vessels in the living body, raius arteries having a great diameter of the blood vessel, and the like), as well as in vein grafts modified by tissue engineering or genetic engineering and various types of artificial blood vessels are also enabled. Therefore, combination with a conventional surgical therapy shall permit the development of broad range of the therapeutic strategy.

In other words, the sustained release preparation of the invention is also useful for employing in bypasses (anastomosis) of a vein graft (great saphenous vein or small saphenous vein or brachial vein, or a modified product thereof by tissue engineering or genetic engineering) and a coronary artery or a branched or capillary vessel thereof, bypasses (anastomosis) of an artificial blood vessel graft (Gore-Tex (registered trademark) (PTFE), Dacron or the like, or a modified product thereof by tissue engineering or genetic engineering) and a coronary artery or a branched or capillary vessel thereof, and bypasses (anastomosis) of a biological graft (artery, vein of human, artery, vein derived from an animal, or a modified product thereof by genetic engineering or tissue engineering such as decellularization) and a coronary artery or a branched or capillary vessel thereof.

### [Detailed Description of the Invention]

Examples of the angiogenesis factor which may be used in the present invention include growth factors such as basic fibroblast growth factor (bFGF) and other FGFs, blood vessel endothelial cell growth factor (VEGF), hepatocyte growth factor (HGF), platelet derived growth factor (PDGF), transforming growth factor (TGF) and angiopoietin, and hypoxia-inducible factor (HIF) having an activity to induce the expression of VEGF and the like, as well as cytokine, chemokine, adrenomeduline and the like having an angiogenetic action.

In the invention, preferable angiogenesis factor is bFGF. The bFGF is a growth factor first identified from pituitary gland and brain as a factor that stimulates proliferation of a fibroblast (3T3) (Rifkin and Moscatelli, J. Cell Biol. 109, 1-6, 1989). It was found that bFGFs have a large variety of functions such as angiogenesis, smooth muscle cell growth, oligophagous therapy, tissue repair, hematopoiesis and nerve cell differentiation in various tissues and organs (Bikfalvi, et al., Endocrine Rev., 18, 26-45. 1997). The bFGFs have been used in Europe and the United States for angiogenesis therapies of ischemic heart diseases at a clinical therapeutic level. In Japan, it has been already commercialized as a skin ulcer therapeutic drug in the field of dermatology, while it has been used in clinical treatments in the field of orthopedic surgery.

Gelatin used in the invention can be obtained from collagen which can be collected from any sites of bodies such as skin, bone and tendon of any of various animal spices including cattle, pig, fishes and the like, or a substance which has been used as collagen, through denaturation by any of a variety of treatments such as alkali hydrolysis, acid hydrolysis and enzymatic decomposition. Denatured gelatin of recombinant type collagen may also be used. Properties of gelatin may vary depending on the materials and treatment processes employed, however, gelatin having any of the properties can be utilized as a hydrogel material for sustained release of the angiogenesis factor such as bFGF in the invention.

Examples of measures representing the properties of gelatin include e.g., isoelectric point, molecular weight, zeta potential and the like. Zeta potential is a measure representing the extent of an electrostatic charge of the substance (gelatin). Particularly preferable gelatin for use in the invention is gelatin having the physical properties described below: available by an alkali hydrolysis treatment from collagen, being acidic gelatin, having the molecule weight of about 100- to about 200-thousand Dalton on an SDS-PAGE under a nonreducing condition, with zeta potential in an aqueous solution being about -15 to about -20 mV. Additionally, acidic gelatin prepared by an alkali treatment of type I collagen derived from bovine bones can be preferably used, alternatively, it is also available as a sample having an isoelectric point (IEP) of 5. 0 of Nitta Gelatin Inc. For reference, basic gelatin prepared by an acid treatment is similarly available as a sample having IEP of 9.0 of Nitta Gelatin Inc., however, the zeta potential greatly varies as follows: acidic gelatin (Nitta Gelatin Inc. , sample IEP 5.0) : about -15 to about -20 mV, basic gelatin (Nitta Gelatin Inc. , sample IEP 9.0): about +12 to about +15 mV.

The gelatin hydrogel for use in the invention means the hydrogel obtained by forming a chemical crosslinkage between any of a variety of chemical crosslinking agents and a gelatin molecule using the aforementioned gelatin. Examples of the chemical crosslinking agent include e.g., glutaraldehyde, water soluble carbodiimide such as EDC, condensing agents which form a chemical bond with propyleneoxide, a diepoxy compound, a hydroxyl group, a carboxyl group, an amino group, a thiol group, an imidazole group or the like. Glutaraldehyde is preferred. Also, gelatin can be chemically crosslinked with a heat treatment, ultraviolet ray irradiation, radiation irradiation, electron beam irradiation or the like. In addition, these crosslinking treatments may be used in combination. Furthermore, the hydrogel can be also produced by physical crosslinking utilizing salt crosslinking, electrostatic interaction, hydrogen bond, hydrophobic interaction or the like.

Degree of crosslinking of the gelatin can be selected ad libitum depending on desired percentage of moisture content, i.e., the level of bioabsorptivity of the hydrogel. Preferred range of concentration of the gelatin and the crosslinking agent upon preparation of the gelatin hydrogel is the gelatin concentration of 1 to 20 w/w% and the crosslinking agent concentration of 0.01 to 1 w/w%. Although the crosslinking reaction condition is not particularly limited, it can be carried out for example, at 0 to 40°C for 1 to 48 hours. In general, as the concentration of the gelatin and crosslinking agent, and crosslinking time period become greater, the degree of crosslinking of the hydrogel increases, leading to reduction in bioabsorptivity.

Although shape of the gelatin hydrogel is not particularly limited as long as it is suited for use in therapy, for example, examples of the shape include sheet form, cylindrical form, prismatic form, discal form, paste form, spherical form, particulate form and the like.

The gelatin hydrogel having a shape of cylindrical form, prismatic form, sheet form or discal form can be prepared by adding an aqueous solution of a crosslinking agent to an aqueous solution of gelatin or adding gelatin to an aqueous solution of a crosslinking agent, followed by pouring the mixture into a mold having a desired shape and allowing a crosslinking reaction to proceed. Alternatively, an aqueous solution of a crosslinking agent may be added to an aqueous solution of a crosslinking agent to a formed gelatin gel neat or after drying. For allowing the crosslinking reaction to be terminated, the reaction mixture is brought into contact with a low molecular weight substance having an amino group such as ethanolamine or glycine, or thereto is added an aqueous solution having the pH of 2.5 or less. For the purpose of completely removing the crosslinking agent and the low molecular weight substance used in the reaction, the resulting gelatin hydrogel is washed with distilled water, ethanol, 2-propanol, acetone or the like, and is subjected to preparation of the formulation.

The gelatin hydrogel having a particulate shape can be prepared by: placing a gelatin solution into an apparatus in which, for example, a motor for stirring (for example, manufactured by Shinto Scientific Co., Ltd., Three One Motor, EYELA miniD. C. Stirrer and the like) fixed to a three-neck round bottom flask with a propeller for Teflon (registered trademark) attached to fix with the flask; adding thereto an oil such as olive oil; stirring at a rate of approximately 200 to 600 rpm to give a W/O emulsion; and adding thereto an aqueous solution of a crosslinking agent, or alternatively, can be prepared by: adding a fluid which had been prepared by preemulsification of an aqueous solution of gelatin in olive oil (using for example, a vortex mixer Advantec TME-21, a homogenizer, polytron PT10-35 or the like) into olive oil dropwise to prepare a finely particulated W/O emulsion, and thereto adding an aqueous solution of a crosslinking agent to allow a crosslinking reaction, followed by recovering the gelatin hydrogel by way of centrifugal separation in either case, thereafter washing with acetone, ethyl acetate or the like, and terminating the crosslinking reaction by immersing in 2-propanol, ethanol or the like. Thus resulting gelatin hydrogel particles are successively washed with distilled water containing 2-propanol and Tween80, and then distilled water or the like, and then subject to preparation of the formulation. When the gelatin hydrogel particles are aggregated, for example, addition of a surfactant or the like, or sonication (preferably, within one minute while cooling) may be carried out.

Mean particle diameter of the resulting gelatin hydrogel particle varies depending on gelatin concentration, volume ratio of the aqueous gelatin solution and olive oil, stirring rate and the like during production of the aforementioned particles. In general, the particles have a diameter of 1 to 1000 µm, and they may be used after sieving to give required size depending on the purpose ad libitum. In addition, preemulsification makes it possible to obtain the gelatin hydrogel having a particulate form with a particle size of 20 micron or less.

Examples of alternative method for preparing the gelatin hydrogel having a spherical shape or a particulate shape also include the following methods. To an apparatus which is similar to that described in the above method is placed olive oil followed by stirring at a rate of approximately 200 to 600 rpm. Thereto is added an aqueous solution of gelatin dropwise to prepare a W/O emulsion. After cooling this emulsion, acetone, ethyl acetate or the like is added and stirred, and uncrosslinked gelatin particles are recovered by centrifugal separation. The recovered gelatin particles are washed with acetone, ethyl acetate or the like, and then with 2-propanol, ethanol or the like followed by drying. Thus dried gelatin particles are suspended in an aqueous solution of a crosslinking agent containing 0.1% Tween80, stirred gently to allow a crosslinking reaction, and washed with a 100 mM glycine aqueous solution containing 0.1% Tween80, 0.004 N HCl containing 0.1% Tween80 or the like depending on the used crosslinking agent, thereby terminating the crosslinking reaction to prepare the gelatin hydrogel particle. Mean particle diameter of the gelatin hydrogel particle obtained according to this method is similar to that obtained in the method as described above.

The gelatin hydrogel of the invention can be used ad libitum after cutting into adequate size and shape, freeze-drying and sterilization. The freeze-drying can be carried out by, for example, placing the gelatin hydrogel into distilled water, freezing in liquid nitrogen for 30 minutes or more, or at -80°C for one hour or more, and drying in a lyophilizer for 1 to 3 days.

Hereinafter, the method of production and the method of use of the angiogenesis factor-containing sustained release preparation of the invention is explained with reference to bFGF as an example. Method of production and use of the sustained release preparation of the invention using an angiogenesis factor other than bFGFs will be apparent based on the disclosure herein and techniques well known in the art.

The bFGF for use in the invention may be any one prepared by any of various methods as long as it is purified to the extent enabling use as a medicine. In addition, commercially available product may be also used. In the method of the production of bFGF, for example, the bFGF can be obtained by culturing a primary culture cell or established cell which produces the bFGF, and isolating and purifying the bFGF from the culture supernatant. Alternatively, intended recombinant bFGF can be also obtained by incorporating a gene encoding bFGF into an appropriate vector by a genetic engineering procedure, transforming the resulting vector into a suitable host by insertion, and recovering the intended recombinant bFGF from the culture supernatant of this transformant. (See, for example, Nature, 342, 440 (1989), JP-A-H5-111382, Biochem. Biophys. Res. Commun. 163, 967 (1989) and the like). The host cell is not particularly limited but various kinds of host cells which have been conventionally used in genetic engineering procedures, for example, Escherichia coli, yeast, insect, silkworm, animal cells and the like can be used. Thus resulting bFGF may include substitution, deletion and/or addition of one or more amino acids in the amino acid sequence as long as it has an action that is substantially similar to naturally occurring bFGF, and may also include substitution, deletion and/or addition of a carbohydrate chain.

The bFGF-containing sustained release gelatin hydrogel preparation of the invention can be obtained by, for example, adding the bFGF dropwise onto the freeze-dried gelatin hydrogel described above, and impregnating the bFGF into the hydrogel. This impregnation operation is usually completed at 4 to 37°C for 15 minutes to 1 hour, preferably, at 4 to 25°C for 15 to 30 minutes. During this operation, the hydrogel is swelled with the bFGF, and the bFGF is immobilized within the gelatin hydrogel by a physical interaction through interacting with the gelatin molecule to form a complex with the gelatin molecule. Not only an electrostatic interaction between the bFGF and gelatin, but also other interactions such as hydrophobic bond and hydrogen bond are believed to be greatly responsible for complex formation of the both substances.

It is preferred that weight ratio of the bFGF to gelatin be about 5 times or less. More preferably, the bFGF is preferably about 5 to about 1/10⁴ times of gelatin by the weight ratio.

The bFGF sustained release gelatin hydrogel preparation of the invention can exert a bFGF function in a small amount for a long period of time because it has a sustained releasing effect and stabilizing effect of the bFGF. Therefore, bio-bypass is formed by effectively exerting the function to stimulate angiogenesis by the bFGF at an administered site, thereby enabling efficacious action as a therapeutic agent for coronary artery narrowing or blockage.

The mechanism of this sustained release is based on physical immobilization of the bFGF on the gelatin within the hydrogel. The present inventors have attempted sustained release of growth factors, cytokine, monokine, lymphokine, other bioactive substances and the like using a bioabsorbable polymer hydrogel so far, whereby having succeeded in sustained release of active ingredients having a bioactivity and in controlling the period of sustained release, both of which have not been achieved by any other material. According to the invention, it is believed that the bFGF is gradually released from the hydrogel by a similar mechanism. In the state of being immobilized on the gelatin hydrogel, the bFGF is hardly released from the hydrogel. As the hydrogel is decomposed in a living body, gelatin molecules become water soluble, accompanied by release of the bFGF immobilized on the gelatin molecule. In other words, decomposition of the hydrogel can control the sustained releasing property of the bFGF. Decomposition property of the hydrogel can be altered by regulating the degree of crosslinking upon production of the hydrogel. In addition, interaction of the bFGF with gelatin improves stability thereof, for example, resistance to enzyme degradation in the living body.

Percentage of moisture content of the gelatin hydrogel of the invention greatly influences on the sustained release, and the percentage of moisture content which presents preferable sustained release effect may be about 80 to 99 w/w%. More preferred percentage is about 95 to 98 w/w%. A measurable marker of this degree of crosslinking includes the percentage of moisture content. Greater percentage of moisture content lowers the degree of crosslinking, thereby facilitating decomposition. Accordingly, the value of this percentage of moisture content exerts an influence on sustained release (gradual release) of the bFGF.

The sustained release preparation of the invention may include a gene encoding an angiogenesis factor. Such a gene is used in the form capable of performing the function within the cell by being introduced into a cell. For example, it is used as a plasmid constructed such that the DNA is transcribed in the cell introduced, and the encoded angiogenesis factor is expressed. Preferably, a promoter region, an initiation codon, a DNA encoding a protein having a desired function, a termination codon and a terminator region are serially arranged.

Such a plasmid can be prepared by inserting a gene that encodes a desired angiogenesis factor into any of various plasmids that are available in this technical field through utilizing an appropriate restriction enzyme site. Also, such a plasmid can be prepared by a synthetic or semi-synthetic means based on the base sequence of the gene to be introduced.

The gelatin hydrogel including a gene that encodes an angiogenesis factor can be produced by a similar method to that described for an angiogenesis factor that is a protein. For example, it can be prepared through: directly immersing a crosslinked gelatin gel prepared by directly adding a crosslinking agent into a 5 to 30% by weight aqueous solution of gelatin or a crosslinked gelatin gel prepared by immersing a uncrosslinked gelatin gel into an aqueous solution of a crosslinking agent into a solution containing a gene; or drying a crosslinked gelatin gel followed by allowing it to be swelled again in a solution containing the gene. Because a nucleic acid is negatively charged, use of a positively charged gelatin hydrogel is preferred.

The sustained release preparation of the invention can be administered by injection or indwelling by a surgical operation in the vicinity of a coronary artery. Specifically, it can be indwelled or administered between a gastroepiploic artery or a greater omentum and a coronary artery, between a left or right internal thoracic artery and a coronary artery, between a left or right radial artery and a coronary artery, or the like. As the sustained release preparation of the invention is gradually decomposed in a living body, the angiogenesis factor is released, and thus, a new blood vessel is induced by the action of the angiogenesis factor between such a blood vessel and a coronary artery or between a branched or a capillary vessel thereof, thereby forming a bio-bypass. Accordingly, blood flow to an ischemic heart is increased, and an effective therapy of coronary artery narrowing or blockage can be achieved.

Alternatively, when a coronary artery bypass surgery is carried out using a venous graft (great saphenous vein or small saphenous vein or brachial vein, or a modified product thereof by tissue engineering or genetic engineering), an artificial blood vessel graft (a modified product thereof by tissue engineering or genetic engineering with Gore-Tex (registered trademark) (PTFE) or Dacron or the like), or a biological graft (artery, vein of human, artery, vein derived from an animal, or a modified product thereof by genetic engineering or tissue engineering such as decellularization) or the like, the sustained release preparation of the invention can be indwelled at the lesioned site or in the vicinity thereof. Also in this instance, the angiogenesis factor is released as the sustained release preparation of the invention is gradually decomposed in the living body. Therefore, action of the angiogenesis factor induces a new blood vessel between the graft and the coronary artery or a branched or capillary vessel thereof to form a bio-bypass thereby increasing the blood flow. Furthermore, the sustained release preparation of the invention can be effectively used also in a bypass surgery where an artificial blood vessel graft and a cell transplantation (myocardial cell, skeletal myoblast, bone marrow cell or cell derived from bone marrow, marrow mononuclear leukocyte cell , ES cell and cells differentiated therefrom, and the like) are used.

Entirety of contents of all patents and cited documents explicitly referred to in this specification is incorporated herein by reference. In addition, entirety of contents described in the specification and drawings of Japanese Patent Application No. 2002-279058 that is a basic application on which this application claims priority is incorporated herein by reference.

### Examples

Hereinafter, the present invention is explained in more detail by way of Examples, however, any limitation is not imposed to the invention by these Examples.

### Example 1. Production of bFGF-containing gelatin hydrogel

After subjecting acidic gelatin having a concentration of 10 wt% to chemical crosslinking by glutaraldehyde, the crosslinking agent was inactivated. Next, the product was washed with distilled water several times to obtain a crosslinked gelatin hydrogel having a percentage of moisture content of 90.3%. Subsequently, a phosphate buffer (pH 7.4, PBS 500 µl) having a concentration of 0.05 M containing 100 pg of bFGF was added dropwise. Accordingly, the bFGF-impregnated gelatin hydrogel was obtained by allowing the aqueous bFGF solution to infiltrate into the gelatin hydrogel.

### Example 2. Production and therapy of myocardial infarction rabbit model

Using Japanese white rabbit weighing 2.5 to 3.5 kg, the heart was exposed by median sternotomy under general anesthesia. While administering an antiarrhythmic drug, a branch of a coronary artery (circumflex) was ligated to produce a myocardial infarction model. Because there may be a case to result in ventricular fibrillation after the surgical operation, the condition was observed for about 20 minutes to await stabilization of the state.

Next, a therapy with the bFGF-containing gelatin hydrogel was conducted to these myocardial infarction rabbit models.
Group A (n=5): untreated (production of myocardial infarction alone).
Group B (n=10): abdominal section conducted, and the collected right gastroepiploic artery including greater omentum folded around the heart.
Group C (n=10): sheet form bFGF-containing gelatin hydrogel administered between right gastroepiploic artery and heart.

Administration of the bFGF was carried out by fixing a hydrogel sheet containing the bFGF on the surface of the heart with a fine thread. Under these conditions, the bFGF is topically released in a sustained manner for about 2 weeks.

### Example 3. Evaluation of cardiac function by ultrasonic examination

Evaluation of the cardiac function was performed by ultrasonic examination of prior to the operation, at 2 weeks, and at 4 weeks. Under mild sedation, body surface ultrasonic examination was performed. Any group exhibited satisfactory cardiac functions prior to the surgery, with no difference existed. At 2 weeks and 4 weeks, the heart contraction ability was significantly satisfactory in the group C to reveal that the cardiac functions were kept.

### Example 4. Evaluation by greater omentum artery imaging examination

At 4 weeks following the operation, imaging examination of the greater omentum was conducted for the groups B and C each consisting of 6 animals, respectively. Anti-coagulation was executed under general anesthesia, and abdominal section was conducted again. Into the greater omentum artery was inserted a 24 G catheter. The animal was sacrificed, and then the greater omentum and the heart were drawn as one block. A contrast medium was infused from the catheter which had been inserted beforehand. After photographing this, the developed film was analyzed.

In group B, coronary artery was visualized from greater omentum artery in two among 6 animals, however, growth of the new blood vessel was very poor. On the other hand, in group C, communication to the coronary artery was found in all 6 animals, and the new blood vessels were extremely rich, all of which were found to communicate with the coronary artery.

### Example 5. Evaluation by histological examination

After the therapeutic period of 4 weeks, the heart specimen was removed, and the region of myocardial infarction was histologically studied. Also, immunological staining was conducted, and number of capillary vessels at the infarction boundary site was determined and subjected to comparative study. The removed heart was sliced horizontally to the long axis of the heart at a part positioned 2 mm far from the coronary artery ligated site toward the apex. The specimen was stained with Masson trichrome to calculate the myocardial infarction region occupying in the entire left ventricle.

Infarction region in the group C was significantly smaller compared to those in groups A and B. Number of arteriola per unit area according to immunostaining (staining of α actin smooth muscular cells of a blood vessel wall) was significantly increased in group C compared to those in other 2 groups.

From the foregoing results, exacerbation of the cardiac function could be significantly prevented, and the myocardial infarction region could be reduced through connection of the blood vessel outside of the heart to the coronary artery (bio-bypass) owing to angiogenetic action by the bFGF-containing gelatin hydrogel, thereby achieving improvement of blood circulation of an ischemic heart.

## Claims

1. A pharmaceutical composition for therapy of coronary artery narrowing or blockage, which comprises a sustained release preparation containing an angiogenesis factor or a gene encoding the same and a gelatin hydrogel.

2. The pharmaceutical composition according to claim 1 wherein said angiogenesis factor is bFGF.

3. A method for treating coronary artery narrowing or blockage comprising administering to a patient a sustained release preparation containing an angiogenesis factor or a gene encoding the same and a gelatin hydrogel.

4. The method according to claim 3 wherein said angiogenesis factor is bFGF.
